# EUROPEAN PATENT APPLICATION

(11) **EP 4 439 455 A2**
(43) Date of publication of application: **02.10.2024**
(21) Application number: 24174044.8
(22) Date of filing: 20.04.2022
(51) Int. Cl.: G06T 7/00

(54) **SYSTEM AND METHOD TO CHARACTERISE A TISSUE ENVIRONMENT IN AN IMAGE**

(30) Priority: 21.04.2021 GB 202105700
(62) Divisional of application: 22720053.2
(71) Applicant: Volpara Health Technologies Limited, Wellington 6011 (NZ)
(72) Inventor: Highnam, Ralph, 6011 Wellington (NZ); Chan, Ariane, 6011 Wellington (NZ)
(74) Representative: Kenny, Andrew

(57) **Abstract**

The present invention relates to the characterisation of a tissue environment within a medical image, for example a quantitative image of a breast. A method is presented of characterization of a tissue environment within a quantitative medical image of a breast to predict risk of benign or malignant lesions via 'blinded' associations. A method is also presented of characterization of a tissue environment within a quantitative medical image of a breast to predict risk of benign or malignant lesions, diagnostic biopsy outcomes or risk of lesion progression, via informed associations of a biopsy or lesion.

## Description

### Field of the Invention

The present invention relates to the characterisation of a tissue environment within a medical image, for example a quantitative image of a breast.

### Background

The human breast is made up of different types of tissue : adipose (or fatty) tissue ; fibrous (or supportive and connective) tissue : and glandular or epithelial tissue (which includes the functional lobular and ductal structures within the breast). Fibrous and glandular tissue are referred to together as 'fibroglandular' tissue (or 'dense tissue'), and their ratio as 'breast density'.

Adipose tissue and fibroglandular tissue can be segmented and quantified in medical images based on their different properties. For example, the difference in x-ray attenuation between adipose and fibroglandular tissues is directly related to the pixel intensities on full-field digital mammography or digital breast tomosynthesis images : adipose tissue has a lower attenuation allowing more x-ray signal to reach the detector ; fibroglandular tissue has a higher attenuation allowing less x-ray signal to reach the detector. This allows for quantification of breast tissue composition at the pixel-level, and subsequent derivation of whole-breast, spatial, textural, and localised characterisation of tissue composition. Another example is the voxel-level segmentation of adipose and fibroglandular tissue on magnetic resonance imaging (MRI), based on the signal that arises from free water molecules or fat-water decomposition.

It is recognised that breast cancers arise predominantly within the glandular tissue and certain breast quadrants such as the upper outer quadrant have higher reported rates of breast cancer. The amount and the proportion of fibroglandular tissue in the breast are strong, independent risk factors for the development of breast cancer: a meta-analysis has shown a 4-6 fold increased likelihood of breast cancer among women with extremely dense breasts (category 'd' under the 'a - d' breast composition categories of the Breast Imaging Reporting and Data System (BI-RADSO)) compared to women with the lowest level of density (BI-RADS category 'a').

Furthermore, as both fibroglandular tissue and lesions appear white and opaque on a mammogram, fibroglandular tissue as a radiological feature, can hide or 'mask' cancers due to tissue superimposition. This increases the risk of breast cancers not being detected during routine screening and/or being detected at a later stage. Interval cancers (such term to mean a cancer that is diagnosed following a negative result from a screening examination, but before the next scheduled screen) are more common in women with increased breast density and tend to be larger and have poorer prognostic outcomes. Although increased breast density is strongly associated with the risk of interval cancers, stratification by breast density suggests that interval cancers diagnosed in women with lower breast density have the most aggressive phenotype.

At screening, the propensity of lesions to develop within fibroglandular tissue has been a factor in identifying specific regions for diagnostic procedures such as biopsy and is a contributing factor to women with increased breast density tending to have higher recall and biopsy rates. Women with a prior biopsy and/or diagnosis of certain benign lesions are also at increased risk of developing breast cancer. Atypical lobular and ductal hyperplasias, for example, are considered to be high-risk lesions, pre-cursor, or pre-malignant lesions that themselves can progress to a malignancy, or are markers for the risk of a concurrent or future breast cancer diagnosis in the ipsilateral or contralateral breast. Histopathological quantification of the epithelial and stromal components of fibroglandular tissue and breast density, have been shown to be independently and jointly associated with risk of subsequent invasive cancer among women with benign breast disease, especially non-proliferative disease.

Furthermore, tissue characterisation may provide predictive information specific to different breast cancer or benign lesion subtypes. It has been shown, for example, that both dense tissue and non-dense tissue are differentially associated with breast cancer subtypes. For example, compared to women with luminal A breast cancers, higher fibroglandular tissue volumes have shown positive associations with human epidermal growth factor receptor-2 (HER2)-positive, luminal B/HER2-negative and luminal B/HER2-positive subtypes. Conversely, 'triple negative' breast cancers are associated with smaller breast volumes and significantly lower non-dense volumes. Triple negative breast cancers account for 10-20% of breast cancers (triple negative breast cancers test negative for oestrogen- and progesterone-receptors, and HER2) and do not respond to targeted therapies, requiring chemotherapy as the primary treatment.

Characterisation of the tissue environment may provide additional predictive or prognostic information in the prevention and adjuvant settings. There is growing evidence that increased breast density is associated with both local and locoregional recurrence. Breast density change has also shown promise as a biomarker for predicting risk-reducing benefits of certain endocrine therapies, such as selective oestrogen receptor modulators. Moreover, breast density may help to predict breast cancer survival, with evidence showing that compared to lower breast density, increased breast density was associated with improved or reduced survival in women who did versus did not receive radiation therapy, respectively.

Thus, differences in, for example, the hormonal milieu, tissue environment and localised peri-lesional micro-environment of the breast play an important role in the heterogeneity, development, and progression of breast cancer. The term 'environment' is used here to refer to the complex of chemical, physical and biological factors that affect an organism or tissue. The term 'microenvironment' is used here to refer to the cells of benign or malignant lesions and the local tissue that those cells interact with or are influenced by, including surrounding stromal cells, extracellular matrix and signaling factors.

Both adipose and fibroglandular tissue are important for predicting potential disease progression. For example, adipose tissue, especially in postmenopausal women, is a key source of oestrogen production through the conversion of androgens by aromatase, which can drive tumour growth. Increased breast density is positively correlated with the abundance of aligned periductal collagen fibrils, which may trigger mechanotransduction pathways that increase tissue stiffness, promote invasion, and further perpetuate an increase in breast density.

Studies have compared the relative significance of localised breast density to global breast density measures. The term `global breast density' is used here to refer to the measures of the percentage or absolute volume of fibroglandular tissue of the whole breast. The term 'localised' is used here to refer to a given region of interest of a specified size or shape, that may or may not incorporate spatial information.

In commercially available density estimation tools, quantitative pixel-level information on breast tissue composition can be further interrogated based on pixel intensity thresholds, and/or spatial and localised information. For example, although the automated density assessment tool Volpara^{®} is routinely used to assess global breast density, quantitative analysis of breast composition based on refined pixel intensity thresholds has shown improved prediction of breast cancer risk. Furthermore, studies have found that global breast density may not adequately capture the risk of benign and malignant lesions, where localised changes are occurring due to complex interactions between the tissue environment and lesion microenvironment.

Machine learning approaches have been used to identify histologic correlates that may underlie breast density and distinguish cancer among women with both high and low breast density. Among the findings are that non-fatty stroma, fat tissue quantities and epithelial region organisation predict fibroglandular volume. Other computational models have been used to determine global and local breast density for intra-operative use to estimate risk of cancer recurrence. Some studies have suggested that beyond the dense tissue percentage, risk prediction can be improved via relative geometric and statistical features of dense tissue over the breast and histograms of standardised local texture features based on sliding windows scanning the whole breast.

Improved means are needed to characterise the breast tissue environment at both a global (i.e. whole breast) and localised level, such that models incorporating image-based tissue characterisation can be developed to help predict for example: whether a benign or malignant lesion (and specific subtypes) might develop within the breast; whether a benign or malignant lesion (and specific subtypes) might develop at a specific location or locations within the breast.; biopsy outcomes for suspicious lesions whereby low-risk lesions can be monitored without requiring further diagnostic workup including biopsy ; which benign lesions are likely to progress to a malignant lesion; which malignant lesions are likely to progress ; and/or response to treatments or risk-reducing medications.

Such models can improve risk stratification and inform decisions around the appropriate screening or management protocols.

Furthermore it would be useful to provide improved means to better predict breast cancer subtypes ; means to determine which benign lesions are more likely to develop into a malignant lesion ; means to characterise the breast tissue environment to improve risk prediction; and means to determine predictors of prognosis.

The present invention provides such means.

### Summary of the invention

According to a first aspect there is a system and method of characterization of a tissue environment within a quantitative medical image of a breast to predict risk of benign or malignant lesions via 'blinded' associations; comprising:
using at least one global density metric of the breast to characterise the tissue environment and a plurality of regions of interest in the tissue environment; and
using a localized quantitative metric based on a density map to rank the regions of interest; determining a tissue characterization map of breast whereon the regions of interest which are within a range of rank are overlaid; and indicating clusters of the regions of interest on the tissue characterization map..

According to a second aspect there is a system and method of characterization of a tissue environment within a quantitative medical image of a breast to predict risk of benign or malignant lesions, diagnostic biopsy outcomes or risk of lesion progression, via informed associations of a biopsy or lesion; comprising:
using at least one global density metric which is characteristic of the tissue environment of the breast and a plurality of regions of interest in the tissue environment;
using a first localized quantitative metric within a single localised region of interest centred on and inclusive of the biopsy or lesion location; and
using a second localized quantitative metric of a peri-lesional environment, where the regions of interest are inclusive or exclusive of the informed biopsy or lesion.

Regions of interest which are inclusive or exclusive of the informed biopsy or lesion may be specified. The region of interest may be one or more of: 1) a Lesional region of interest; 2) an offset region of interest (which can be zero mm or greater), that may include or exclude the lesion/lesional ROI; and/or; 3) the peri-lesional region, which may exclude the lesional ROI and any offset.

A system and method to characterise a tissue environment provides means to characterise a tissue environment within a medical image, for example a quantitative image of a breast, whereby it can be more accurately predicted whether an environment is conducive to the development of a benign or malignant lesions. In a preferred embodiment, the environment is characterised using refined measures of volumetric breast density.

The system and method to characterise a tissue environment may provide means for localised metrics and/or a localised map that is improved predictor of an environment in which a cancer might develop. In a preferred embodiment, the environment is characterised using refined measures of volumetric breast density.

The system may comprise a means to acquire and/or analyse a medical image, digitize the medical image.

In particular the system and method to characterise a tissue environment may provide means to characterise the breast tissue environment at a global (i.e. whole breast) and/or a localised level. The global and localized levels of characterization help predict whether a benign or malignant (and specific subtype(s) of) lesion might develop within the breast; whether a benign or malignant lesion (and specific subtype(s)) might develop at one or more specific location(s) within the breast.; the biopsy outcomes for suspicious lesions (whereby low-risk lesions can be monitored without requiring further diagnostic workup (including biopsy)) ; response to treatments or risk-reducing medications.

It is an advantage that system and method to characterise a tissue environment can improve risk stratification and inform decisions around the appropriate screening or management protocols.

The system may comprise a means are to characterise a tissue environment.

Preferably the means are provided to characterise the tissue environment around a lesion.

Preferably the means are provided to characterise the tissue environment before the identification of a lesion, whereby a predictive location of a lesion (benign or malignant) is indicated and preferably before the identification of such a lesion by a clinician, for example a radiologist and/or before any biopsies have been performed.

Preferably the means are provided to characterise the tissue environment whereby density is determined, for example, density around a lesion or a predictive location of a lesion. A means may be provided to determine peri-lesional density relative to global density.

Preferably the means to characterise the tissue environment are provided to determine 'n' regions of a specified characteristic and to determine the relationship between the 'n' regions is determined.

Preferably histopathological findings are included in the criteria to determine 'n' regions.

Preferably localised tissue characterisation is used to determine the effect of an intervention, for example, measures of density, are used to predict possible biopsy clip/wire migration.

Preferably the method and system, and the quantity and magnitude of 'n' regions helps to predict biopsy outcomes (e.g. lobular carcinoma in situ, atypical hyperplasia etc.), or the risk of malignancy of the biopsied lesion or elsewhere in the breast(s).

Preferably the measure of density is refined, for example through thresholding of pixel intensities, whereby imaging biomarkers are derived that indicate the pathogenicity of the tissue environment or lesion micro-environment. The term 'biomarker' is used here to refer to a naturally occurring characteristic by which a particular pathological or physiological process, disease, etc. can be identified.

Preferably localised tissue characterisations are correlated across screening and/or diagnostic mammographic views to more accurately triangulate tissue of interest.

In an embodiment, a tissue characterisation map may be generated to assess the propensity of the breast and/or localised regions within the breast to develop benign and/or malignant lesions and/or determine respective risks thereof.

A number of distinct or overlapping regions of interest in the tissue environment may be identified and ranked based on variable criteria in order to indicate high risk breasts and/or localised regions within the breast.

For example, a region of interest may be identified based on one or more of the following: size of the region(s) of interest ; shape of the region(s) of interest ; percentage or absolute volume of dense or fat tissue of the region of interest ; the average or maximum thickness of the dense or fat tissue within the region of interest.

Higher risk areas may be subsequently indicated based on one or more of the following: the number of regions of interest identified ; comparisons of the region(s) of interest with global breast density metrics ; cumulative percent or absolute volumes of dense or fat tissue summed or averaged across all regions of interest or a subset therein.

In an embodiment the region of interest may comprise adipose tissue.

In an embodiment, regions of interest may be clustered together based on similarity of the regions of interest and/or spatial distribution, whereby the numbers, size, and/or spatial distribution of clusters can provide additional information related to risk of benign or malignant lesions. Regions of interest may be designed to have a dimension of maximum width between 2 mm and 30 mm according to a size of a lesion of interest. The ROI size may be restricted by the dimensions of the compressed breast.

In an embodiment, clustered regions of interest may be used to create a masking map to indicate regions within the breast that are more likely to mask or obscure lesions. For example, clusters of a particular size, spatial distribution, and/or with a particular density could indicate the risk of masking of varied lesion sizes or varied lesion presentations (such as solid or spiculated masses or microcalcifications).

In an embodiment the peri-lesional density regions of interest may be used to predict surgical outcomes, for example, peri-lesional density is predictive of surgical outcomes such as re-excision rates.

In an embodiment, tissue characterisation maps may be compared longitudinally across two or more timepoints, to evaluate the consistency in the numbers, ranks, clustering and/or locations of identified regions of interest as an indicator for risk of benign or malignant lesions. Quantitative changes, (such as changes in the percentage or absolute volumes of dense or fat tissue) between longitudinal pairs of localised regions of interest can also inform risk predictions.

In an embodiment, regions of interest may be evaluated across distinct mammographic views (such as craniocaudal, mediolateral oblique, lateral views) to provide more refined spatial and localisation information relating to specific regions, by way of triangulation across the views.

In an embodiment, a region or regions of interest may be indicated (automated, manually, or otherwise interactively) for example by a visual indicator such as the location of a given lesion or lesions and/or biopsy locations. For example, a region or regions of interest could be centred around the location of interest; sampled around and excluding the lesion of interest ; sampled around the lesion incorporating anatomical positions, such as lateral, medial, superior, inferior, anterior, or posterior directionality relative to the lesion. Percentage or absolute volumes of density or fat tissue of the peri-lesional region(s) of interest, or perhaps the peri-lesional density relative to the global breast density could inform risk predictions relating to benign or malignant lesions.

In an embodiment, information from tissue characterisations and global breast density may be combined with histopathological and genomic findings to inform the risk of future benign or malignant lesions developing within the breast(s) and/or the risk of progression of identified benign or malignant lesions.

The invention will now be described, by way of example only, with reference to the accompanying figures in which:

### Brief Description of the Figures

Figure 1 shows determined non-overlapping regions of interest based on density in a first breast;
Figure 2 shows a determined cluster of regions of interest in the first breast;
Figure 3 shows a table with information on the regions of interest in Figure 1;
Figure 4 shows determined non-overlapping regions of interest based on density in a second breast;
Figure 5 shows a determined cluster of regions of interest in the second breast;
Figure 6 shows a table with information on the regions of interest in Figure 4;
Figure 7 shows determined non-overlapping regions of interest based on density in a third breast;
Figure 8 shows several clusters of regions interest in the third breast;
Figure 9 shows a table with information on the regions of interest in Figure 7;
Figure 10 shows determined non-overlapping regions of interest based on density in a fourth breast;
Figure 11 shows two determined clusters of regions interest in the fourth breast;
Figure 12 shows a table with information on the regions of interest in Figure 9;
Figure 13 shows a legion surrounded by an annular peri-lesional region of interest not touching the legion;
Figure 14 shows a legion surrounded by an annular peri-lesional region of interest touching the legion;
Figure 15 shows various shapes and sizes of peri-lesional regions of interest outside of a lesion region of interest;
Figure 16 shows a density map of a fifth breast;
Figure 17 shows a lesion (plus offset) and a peri-lesional region of interest in the fifth breast;
Figure 18 shows a table with information on the regions of interest in Figure 17;
Figure 19 shows a density map of a sixth breast;
Figure 20 shows a first and a second lesion (plus offset) and respective first and second peri-lesional region of interest of the sixth breast; and
Figure 21 shows a table with information on the regions of interest in Figure 20.

### Detailed Description of the Invention

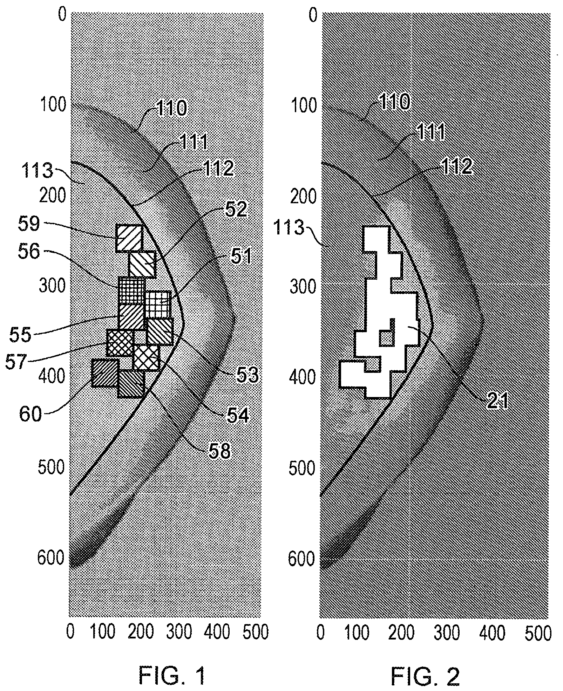

Referring to the Figures, there is shown in Figures 1, 2, 4, 5, 7, 8, and 10, and 11 determination of regions based on density. In these figures the top 10 densest, 10 mm x 10 mm square regions of interest have been identified within the inner breast region.

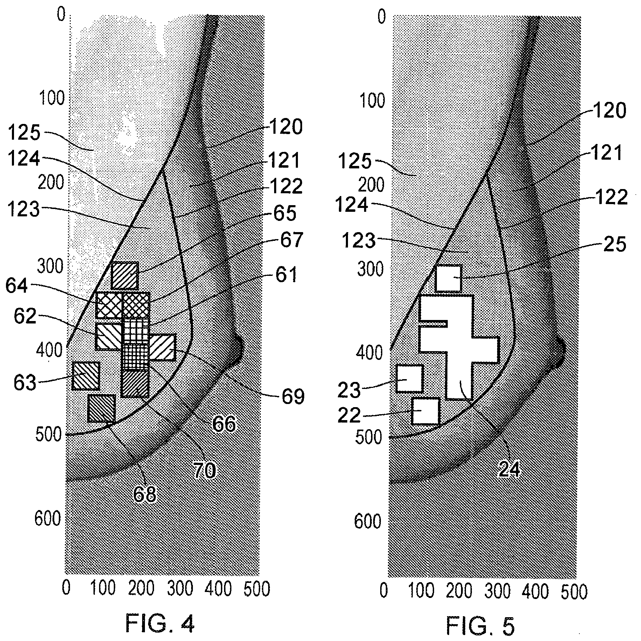

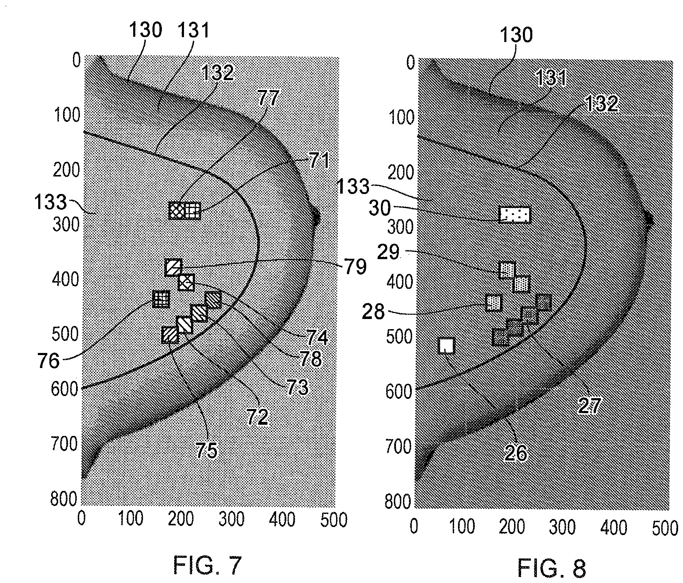

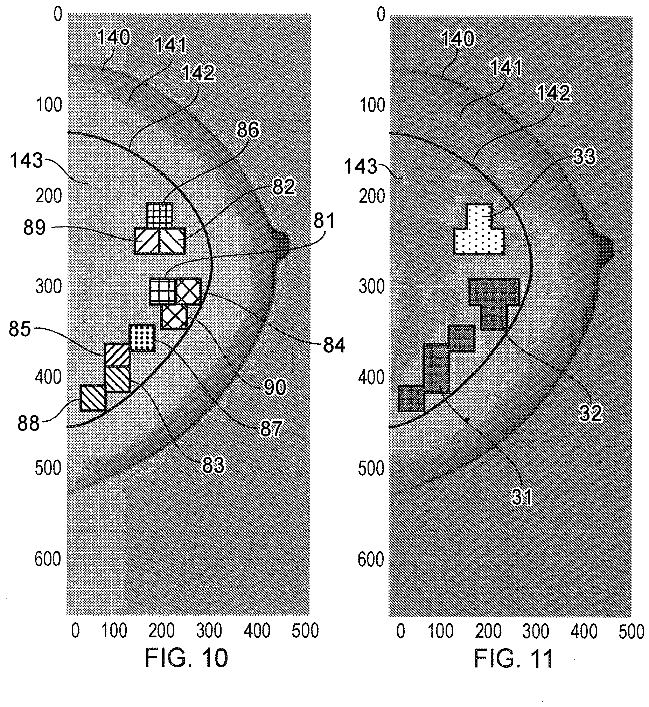

These figures show four example breasts each with a particular global breast density. The global breast density s the ratio of fibroglandular tissue to total breast volume expressed as a percentage. In a first example Figures 1, 2 show a first breast which has a global breast density of 15.71 percent. Figures 4, 5 show a second example in which a second breast has a global breast density of 27.63 percent. Figures 7, 8 show a third example in which a third breast has a global breast density of 3.78 percent. Figures 10, 11 show a fourth example in which a fourth breast has a global breast density of 22.90 percent.

The second breast shown in the second example of Figures 4, 5 has relatively the highest global breast density. The third breast shown in the third example of Figures 4, 5 has relatively the lowest global breast density.

In each of the four example breasts, the skin line 110, 120, 130, 140 demarcates the breast tissue from the background, and the inner breast region 113, 123, 133, 143 is shown inside a demarcation line 112, 122, 132, 142 inwards from the skin line 110, 120, 130, 140. In the inner breast region 113, 123, 133, 144 a constant breast thickness is assumed. In the outer breast region 111, 121, 131, 141 a non-constant breast thickness is assumed. In the second breast there is separation line 124 between segmented pectoral muscle 125 and the inner breast region 123 and outer breast region 121.

In the left-hand image Figure 1, Figure 4, Figure 7, and Figure 10 there is depicted a density map, in which square coded outlines of ten identified, non-overlapping regions of interest are shown in the inner breast region 113, 123, 133, 143. Each region of interest has a square area in the breast of 10 mm x 10 mm. Regions of interest are smaller regions within the inner breast region.

Each of the regions of interest is a column with the square area as its cross section and a length so that each region of interest has volume. The length may be the compressed breast thickness.

In Figure 1 the regions of interest are labeled 51, 52, 53, 54, 55, 56, 57, 58, 59, 60. In Figure 4 the regions of interest are labeled 61, 62, 63, 64, 65, 66, 67, 68, 69, 70. In Figure 7 the regions of interest are labeled 71, 72, 73, 74, 75, 75, 77, 78, 79, 90. In Figure 10 the regions of interest are labeled 81, 82, 83, 84, 85, 86, 87, 88, 89, 90.

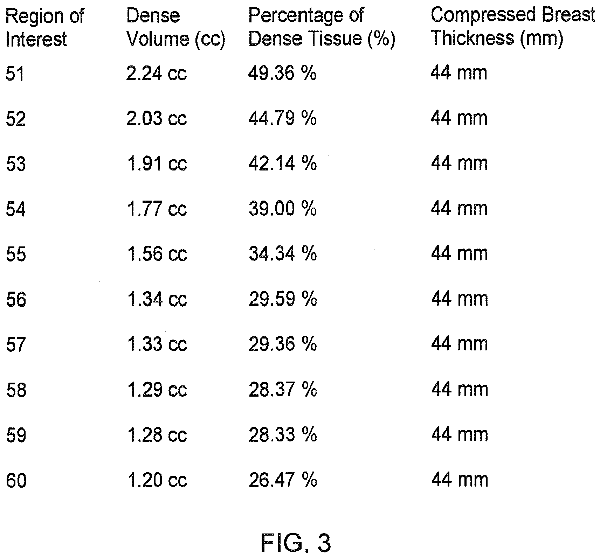

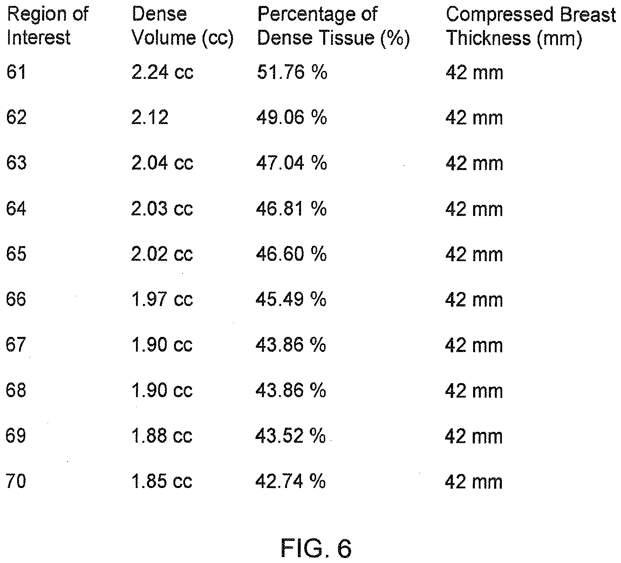

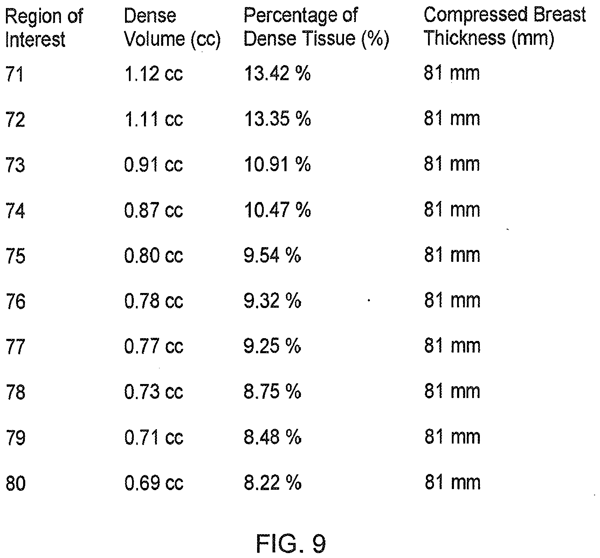

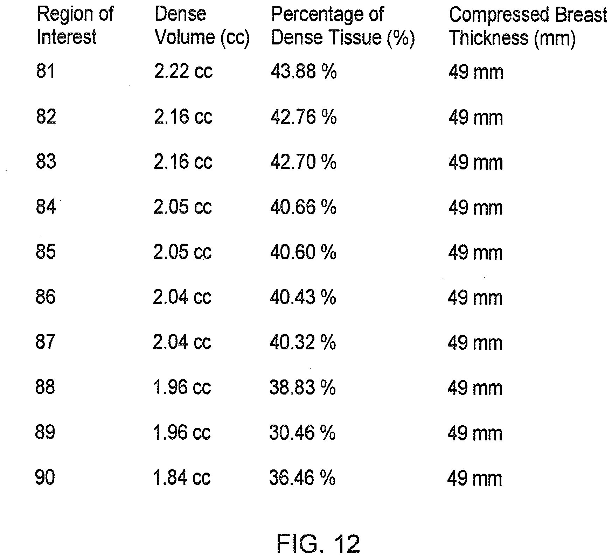

In Figure 3 there is a table with information on the regions of interest in Figure 1. The information includes the absolute volume of dense (i.e. fibroglandular tissue) material in each region of interest. Likewise in each of Figures 6, 9, and 12 there is a table for Figures 4, 7, and 10 respectively.

Each region of interest is ranked in descending order based on both (absolute dense volume in cubic centimetres (cc) and percentage of dense tissue) of the column of tissue specified by the region of interest. For example in Figure 1 the region of interest 51 has an absolute dense volume of 2.24 cubic centimeters (cc) meaning there is 2.24 cc of dense tissue, i.e. fibroglandular tissue. The percentage of dense tissue is 49.36 percent. The compressed beast thickness is 44 mm for region of interest 51.

In each right-hand Figure 2, 5, 8, and 11 there is an image of a density map of the first breast, second breast, third breast, and fourth breast respectively. With each density map a number of clusters is identified (number of clusters = n).

In right hand Figure 2 one cluster 21 is identified (n=1) in the first breast inner breast region 113. The one cluster 21 includes all ten regions of interest 51, 52, 53, 54, 55, 56, 57, 58, 59, 60.

In the right-hand Figure 5 the one cluster 24 is identified which includes seven regions of interest 61, 62, 64, 66, 67, 69, 70 of the second breast. The lone areas 22, 23 and 15 which correspond to regions of interest 68, 63, and 65 are not in any cluster.

The method of characterization of the tissue environment in Figure 4 in some embodiments determines there is one cluster. Then the right hand Figure the once cluster 24 includes individual squares 22, 23 so that the one cluster includes the ten regions of interest 61, 62, 63, 64, 65, 66, 67, 68, 69, 79 of the second breast.

In the right-hand Figure 8 there are three clusters 27, 29, and 30 identified in the inner breast region 133 of the third breast. First cluster 27 includes four regions of interest 72, 73, 75, 78. Second cluster 29 includes two regions of interest 74, 79. Third cluster 30 includes two regions or interest 71, 77. Lone area 28 which corresponds to region of interest 76 is not in any cluster.

The method of characterization of the tissue environment in Figure 7 in some embodiments determines squares 28 and 29 belong in a single cluster. In the right-hand Figure 8 there are three clusters 27, 28 plus 29, and 30 identified in the inner breast region 133 of the third breast. First cluster 27 includes four regions of interest 72, 73, 75, 78. Second cluster 28 plus 29 includes three regions of interest 74, 76, 79. Third cluster 30 includes two regions or interest 71, 77.

Lone area 26 corresponds to a region of interest too faint to be seen in left hand Figure 7.

In the right-hand Figure 11 there are two clusters 31, 33 identified in the inner breast region 143 of the fourth breast. First cluster 31 includes seven regions of interest 81, 83, 84, 85, 87, 88, 90. Second cluster 33 includes regions of interest 82, 86, 89.

The method of characterization of the tissue environment in Figure 10 in some embodiments determines areas 31 and 32 are in a single cluster. In the right-hand Figure 11 there are two clusters 31 plus 32, and 33 identified in the inner breast region 143 of the fourth breast. First cluster 31 plus 32 includes seven regions of interest 81, 83, 84, 85, 87, 88, 90. Second cluster 32 includes regions of interest 82, 86, 89.

In right hand Figure 2, Figure 5, Figure 8, and Figure 11 the number of clusters (n) identified is 1, 1, 3 and 2 respectively. Therefore the number of clusters may be denoted as n=1, n=1, n=3 and n=2 respectively in the first breast, second breast, third breast, and fourth breast respectively.

The clustering method used to find the clusters is Density-Based Spatial Clustering of Applications, DBSCAN.

Thus in an embodiment, and with reference to the figures mentioned above, 'n' regions are determined based for example on density using a density map. Statistical or structural texture measures may be computed. For example texture measures may include statistical measures (standard deviation, variation, gray level co-occurrence matrix (GLCM)) and/or structural measures (edge features e.g. counting number of edges in the region). Texture measures can be taken from the inner breast region 113, 123, 133, 143, (global metric); outer breast region 111, 121, 131, 141; each region of interest 51- 90 (localised metric); cluster of regions of interest 21, 24, 27, 29, 30, 31, 33, and/or a central region of the inner breast (i.e. where breast is estimated to be in contact with the paddle around the central region).

The relationship between locations of 'n' regions is evaluated. For example : if all regions are near or clustered to each other (almost overlapping), then this might indicate a particularly dense region of the breast tissue environment. The particular dense region would have a propensity of benign or malignant lesions to develop.

Patterns of clustering or the identification of multiple, spatially distinct clusters, especially if informed by anatomical positioning, might indicate localised tissue regions within the breast that are at higher risk or provide an indication of the heterogeneity of the breast tissue. It is recognised that breast cancers arise predominantly within the glandular tissue. Certain breast quadrants such as the upper outer quadrant anatomical position have higher reported rates of breast cancer.

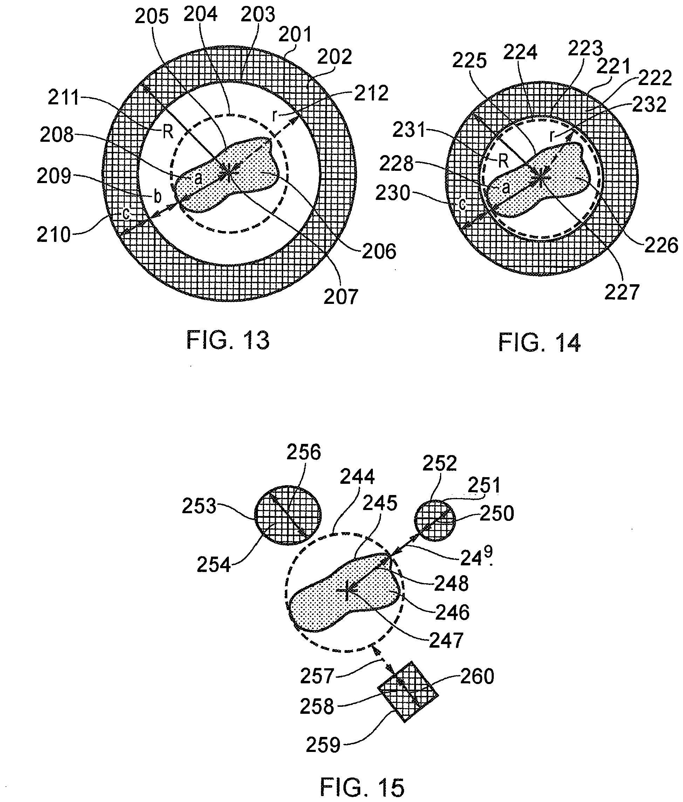

There is shown in Figure 13, Figure 14, and Figure 15 positions of lesions 206, 226, 246 with respect to peri-lesional regions of interest 202, 222, 251, 254, 258. Coordinates, dimensions, and diameters are shown which identify lesion and peri-lesional regions of interest and their respective size and position, inclusive or exclusive of the lesion itself. Figure 13, Figure 14, and Figure 15 each show examples of using known lesion or biopsy coordinates and known lesion radii to automatically identify lesional and peri-lesional regions of interest.

A peri-lesional region or a peri-lesional micro-environment 202, 222 such as shown in Figure 13 and Figure 14 surrounds a lesional region and/or lesional micro-environment 206, 226. The peri-lesional region may be inclusive of the lesional region.

In left-hand Figure 13 there is depicted a lesion 206 and a cross at the lesion centre 207. The depicted lesion 206 is oblong, though this is not necessary. A longest radius 'a' 208 of the lesion is found and taken to be known. The longest radius 'a' 208 may be known from a measurement of the size of the lesion 206 in a density map such as in Figure 1. The longest radius 'a' may be retrieved or predicted from a database gathered previously of lesions of type like oblong lesion 206. Hence the preselected or estimated lesion longest radius 'a' 208 may be said to be 'known' since it is based data or measurement.

The longest radius 'a' 208 is used to demarcate a lesion region of interest 204 (dashed black line) centering around the lesion centre 207. The peri-lesional region of interest 202 (square grid pattern) is an annulus shape. The width 'c' 210 of the annulus shape is greater than zero. The width 'c' 210 is defined by the difference between the larger circle 201 with a larger radius, 'R' 211, and the smaller circle 203 with a smaller radius, 'r' 212. The larger radius 'R' 211 is the sum of the longest lesion radius, 'a' 208 , plus an offset greater than zero, 'b' 209, plus a peri-lesional width 'c' 210 of the annular peri-lesional region of interest 202. The smaller radius 'r' 212 is the sum of the lesion longest radius, 'a' 208 plus an offset equal or greater than zero, 'b' 209.

As shown in the left-hand Figure 13 the lesion 206 is separated from the peri-lesional region of interest 202 by at least the offset 'b' 209. The offset 'b' 209 is greater than zero.

The right-hand Figure 14 differs in that the offset is zero between the smaller circle 223 of the peri-lesional region of interest 222 and the longest radius 'a' 228 of the lesion 226. In the right-hand Figure 14 there is no offset like 'b' 209. The lesion 226 touches the smaller circle 203 of the annulus of the peri-lesional region of interest 223.

The outer limit 224 of the lesion 226 is equal to the smaller circle 223.

In the right-hand Figure 14 the peri-lesional region of interest 222 has an annulus shape which has an outer limit of larger outer circle 221. The lesion 226 has a centre 227 which is also the centre of the peri-lesional region of interest 222. The width 'c' 230 of annulus shape is the difference between larger radius 'R' 231 of the outer circle 221 and the smaller radius 'r' 222 of the inner circle 223.

A peri-lesional region or a peri-lesional micro-environment 251, 254, 258 such as shown in Figure 15 is a proximate region that does not surround a lesional region of interest perimeter 244 micro-environment. The peri-lesional regions 251, 254, 258 are exclusive of a lesion 246. The lesion 246 may be one with informed associations (e.g. with prior knowledge of the biopsy/lesion location and/or size). The lesion 246 may be one with a 'blinded' associations (e.g. without prior knowledge of the biopsy locations).

Figure 15 depicts a lesion 246 and lesion centre 247 (cross). A known lesion radius 248 is used to demarcate the lesion region of interest 244 (dashed black line) centering around the lesion centre 247. The lesion radius 248 is taken to be known from data or measurement as explained above. Examples are provided of peri-lesional regions of interest of varying shapes. There is first peri-lesional region of interest 260 having a square perimeter 259 and a width 260. There is a second peri-lesional region of interest 251 having a smaller circle perimeter 252 and diameter 250. There is a third peri-lesional region of interest 254 having a larger circle perimeter 253 and diameter 256. The first and second peri-lesional regions of interest 251, 258 each have a radial offset 249, 257 of their perimeter 252, 259 and the lesion radius 248 of the circular demarcation 244 of the lesion region of interest 244.

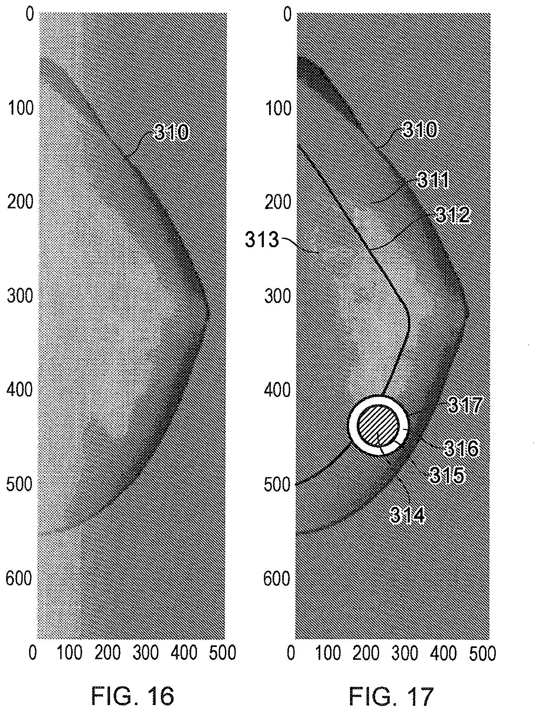

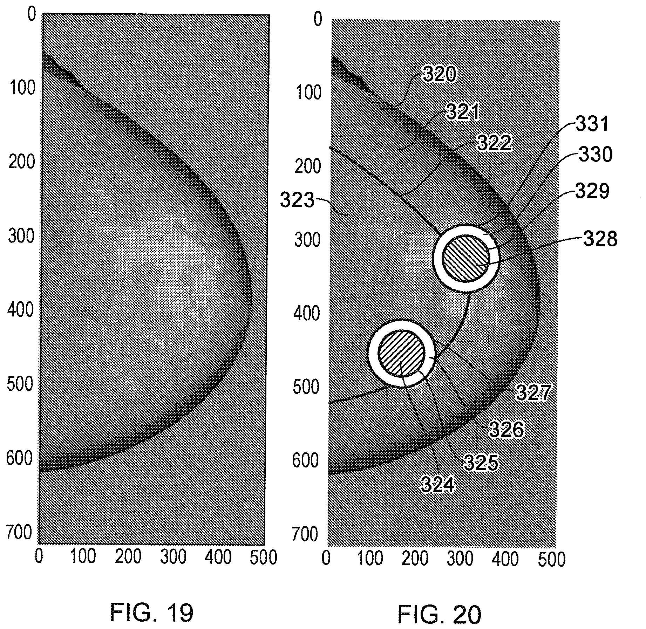

There is shown in Figures 16, 17, 19, and 20 examples of peri-lesional analysis based on varying shapes, sizes, and distances from the center of the lesion or periphery of a lesion region of interest. Figures 16 and 17 show a first example and Figures 19 and 20 show a second example. In the examples one or more known lesion coordinate(s) and diameter(s) are used to identify lesion and peri-lesional regions of interest. They are known from data previously collected or measurement as explained above. As explained with reference to Figures 13 and 14 compared to Figure 15, the lesion and peri-lesional regions of interest may be inclusive or exclusive of the lesion itself.

In the left-hand images of Figure 16 and Figure 19 is depicted a density map. In the right-hand images of Figure 17 and Figure 20 is depicted the lesion regions of interest 314, 324, 328 and peri-lesional regions of interest 316, 326, 329. There is a demarcation line 312, 322 of the inner breast region 313, 323, i.e. where a constant breast thickness is assumed.

In Figure 17 and 20 there is one or more lesion region(s) of interest outer perimeter circle 315, 325, 329 is identified by the known lesion coordinate(s) and a distance from the centre of the lesion(s) that includes the lesion diameter(s) plus a four millimetre offset like 'b' 209 or 249, 257 as explained above for Figure 13 and Figure 15.

In right hand Figure 17 and 20 there are one or more peri-lesional region(s) of interest 316, 326, 330 identified by an annulus shape with an outer circular perimeter 317, 327, 331 surrounding an inner circular perimeter 315, 325, 229 and lesion region(s) of interest 314, 324, 328. The annulus shape of the peri-lesional region of interest 316, 326, 330 has a two millimetre width from inner circle 315, 325, 329 to outer circle 317, 327, 331.

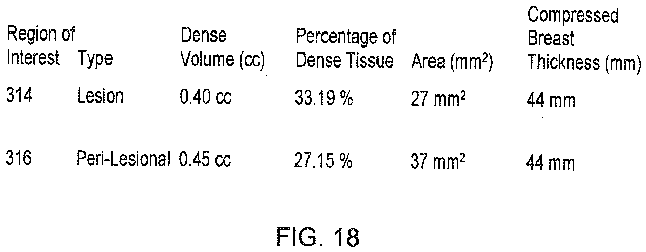

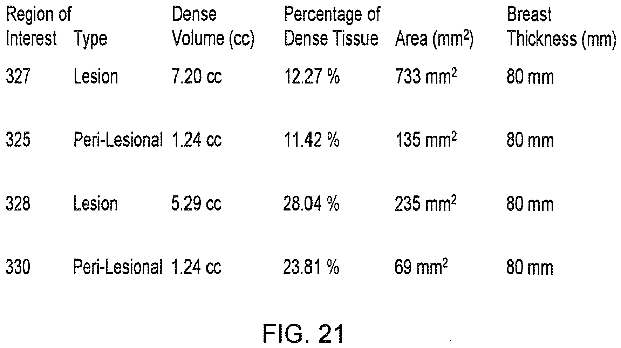

In Figures 18 and 21 there are tables which with information on the legion regions of interest 314 324, 328 and the peri-lesional regions of interest 316, 326, 330. The information includes absolute dense volumes in cubic centimetres, percentages of dense tissue, absolute areas in square millimeters, and compressed breast thickness in millimetres for the lesion (plus offset) and peri-lesional regions of interest separately.

In an embodiment, and with reference to Figures 2 and 3, a lesion location is known along with the diameter of the lesion. An automated peri-lesional assessment of tissue is conducted at a known distance or known distances from the centre of the lesion, using regions of interest of varying shapes and sizes.

In an embodiment, a lesion location is known. Anatomical positions are identified, for example using the laterality and view information in conjunction with breast segmentation software for automated identification of anatomical landmarks such as the nipple, inframammary fold, pectoral muscle. Position information is used to identify localised or peri-lesional regions of interest, for example, in the posterior, anterior, medial, lateral, superior, inferior to a potential lesion location. Distance thresholds are then set : e.g. localised density greater than one centimetre from the centre of the lesion, towards the lateral aspect of the breast etc.

The size of the localised regions can be based on, for example : lesion size if known (e.g. pre-set ranges based on informed knowledge of the reported lesion size or typical lesion sizes) ; a relative to breast area measure (e.g. localised region as 1%, 10%, 25% etc. of total breast area) ; a relative to breast thickness ; and or relative to the estimation of volumetric density.

In an embodiment, the system and method to characterise a tissue environment provides a framework to automate the analysis: develop machine learning algorithms to, for example : learn the most predictive size, shape, thresholds, and numbers of the regions of interest ; learn the most predictive clustering patterns or locations of identified regions of interest; segment out biopsy markers (if any) or lesions.

In an embodiment the tissue characterisation and clustered regions can be used to indicate masking and to create a masking map.

By way of illustrative embodiment the system and method to characterise a tissue environment is used to predict risk of benign or malignant lesions via 'blinded' associations (e.g. without prior knowledge of the biopsy locations).

The system and method to characterise a tissue environment is based on:
a. Global density metrics, e.g. volumetric breast density, volume of fibroglandular tissue, breast volume;
b. refined global density metrics based on varied thresholds, e.g.
   i. percentage volumetric breast density or absolute dense volume considering only pixels that have greater than 6%, 8%, 10%, 15%, or 20%;
   ii. percentage volumetric breast density or absolute dense volume considering only pixels that have greater than 5, 10, 15, or 20 mm of dense tissue thickness;
   iii. percentage area or absolute area considering only pixels that have greater than 6%, 8%, 10%, 15%, or 20%;
   iv. percentage area or absolute area considering only pixels that have greater 5, 10, 15, or 20 mm of dense tissue thickness;
c. localised quantitative metrics based on density maps and identified regions of interest based on:
   i. varying shape, for example squares or circles;
   ii. varying diameter, for example 5 mm (similar to a small lesion) or 30 mm (similar, to a large lesion);
   iii. varying numbers of regions of interest, for example the 10 densest regions of interest, where 'densest' can be defined based on the percentage of dense tissue or absolute dense tissue volume within the column of tissue specified by the region of interest;
d. a tissue characterisation map where regions of interest (ranked in order of density) are overlaid onto the density map and similar regions of interest are clustered.

The system and method to characterise a tissue environment is used to predict risk of benign or malignant lesions, diagnostic biopsy outcomes or risk of lesion progression, via informed associations (e.g. with prior knowledge of the biopsy/lesion location and/or size) based on :
a. global density metrics, e.g. volumetric breast density, volume of fibroglandular tissue, breast volume;
b. percentage of dense tissue or absolute dense tissue volume within a single localised region of interest centred on the biopsy location, where the region(s) of interest is based on:
   i. varying shape, for example squares or circles;
   ii. varying diameter, for example 5 mm (similar to a small lesion) or 30 mm (similar to a large lesion);
c. percentage of dense tissue or absolute dense tissue volume of the peri-lesional environment, where the regions of interest are inclusive or exclusive of the informed lesion, and based on:
   i. varying shape, for example squares or circles;
   ii. varying diameter, for example 5 mm (similar to a small lesion) or 30 mm (similar to a large lesion);
   iii. varying distance from the centre of the informed biopsy location or lesion, for example 10 mm from the centre of the lesion or biopsy location (The centre of the lesion or biopsy location is the centre of the single localised region of interest centred on the biopsy location);
   iv. varying distance from the periphery of the informed lesion, for example 10 mm from the peripheral edge of a lesion derived from a known biopsy location or lesion and known diameter (the distance may be restricted by dimensions of the compressed breast);
   v. varying numbers of regions of interest surrounding the lesion and incorporating anatomical positioning, for example :
      i. eight 10 mm diameter, square regions of interest, in a circular arc surrounding the entire lesion where the centre of each region of interest is 10 mm from the lesion/biopsy centre;
      ii. one 30 mm diameter, circle region of interest anterior to the lesion/biopsy, at a distance of 10 mm from the lesion/biopsy centre;
      iii. two 20 mm diameter, square regions of interest, one anterior and one lateral to the lesion/biopsy, at a distance of 20 mm from the lesion/biopsy centre;
   vi. determination of the relative localised or peri-lesional density (percentage of dense tissue or absolute dense tissue volume) compared to reference metrics, for example:
      i. compared to the global breast density
      ii. compared to similar regions of interest on the contralateral breast.

The invention has been described by way of examples only. Therefore, the foregoing is considered as illustrative only of the principles of the invention. Further, since numerous modifications and changes will readily occur to those skilled in the art, it is not desired to limit the invention to the exact construction and operation shown and described, and accordingly, all suitable modifications and equivalents may be resorted to, falling within the scope of the claims.

## Claims

1. A method of characterization of a tissue environment within a quantitative medical image of a breast to predict risk of benign or malignant lesions, diagnostic biopsy outcomes or risk of lesion progression, via informed associations of a biopsy or lesion; comprising:
using at least one global density metric which is characteristic of the tissue environment of the breast and a plurality of regions of interest in the tissue environment;
using a first localized quantitative metric within a single localised region of interest centred on and inclusive of the biopsy or lesion location; and
using a second localized quantitative metric of a peri-lesional environment, where the regions of interest are inclusive or exclusive of the informed biopsy or lesion.

2. A method of characterization of a tissue environment according to claim 1 wherein the first localized quantitative metric is the percentage of dense tissue volume or area or absolute dense tissue volume or area within the single localised region of interest centred on the biopsy or lesion location.

3. A method of characterization of a tissue environment according to claim 1 comprising designing the single localised region of interest centred on the biopsy or lesion location to have a dimension between 2 mm and 30 mm according to the informed biopsy or lesion or restricted by dimensions of the compressed breast.

4. A method of characterization of a tissue environment according to claim 1 designing the single localised region of interest centred on the biopsy or lesion location to have a size and shape according to the informed biopsy or lesion.

5. A method of characterization of a tissue environment according to claim 1 wherein the second localized quantitative metric is percentage of dense tissue volume or area or absolute dense tissue volume or area of the peri-lesional environment.

6. The method of characterization of a tissue environment according to claim 1 comprising designing the distance in range of 2 mm to 30 mm from the peripheral edge of the informed lesion to the perimeter of each of the regions of interest.

7. The method of characterization of a tissue environment according to claim 1 comprising determining a ratio of global breast density to the localised percentage of dense tissue within the single localised region of interest centred on the biopsy or lesion location.

8. The method of characterization of a tissue environment according to claim 1 comprising determining a ratio of global breast density to localised density within the single localised region of interest centred on the biopsy or lesion location.

9. The method of characterization of a tissue environment according to claim 1 comprising determining a ratio of peri-lesional density to localised density within the single localised region of interest centred on the biopsy or lesion location.

10. The method of characterization of a tissue environment according to claim 1 comprising determining a ratio of global breast density to the peri-lesional density.
